Europäisches Patentamt

European Patent Office

Office européen des brevets

(19)

(11) Veröffentlichungsnummer: **0 267 383 B1**

# EUROPÄISCHE PATENTSCHRIFT

(12)

(45) Veröffentlichungstag der Patentschrift: **16.10.91**

(21) Anmeldenummer: **87112971.4**

(22) Anmeldetag: **04.09.87**

(51) Int. Cl.⁵: **C07C 45/67**, C07C 49/413, B01J 23/10, B01J 23/06, C07C 45/45

(54) **Verfahren zur Herstellung von Cycloheptanon.**

(30) Priorität: **06.11.86 DE 3637787**

(43) Veröffentlichungstag der Anmeldung:
**18.05.88 Patentblatt 88/20**

(45) Bekanntmachung des Hinweises auf die
Patenterteilung:
**16.10.91 Patentblatt 91/42**

(84) Benannte Vertragsstaaten:
**CH DE FR GB IT LI NL**

(56) Entgegenhaltungen:
**DE-A- 1 768 138**

**CHEMICAL ABSTRACTS, Band 98, Nr. 5, 31. Januar 1983, Seite 618, Spalte 1, Zusammenfassung Nr. 34199k, Columbus, Ohio, USA; T. V. VASINA et al: "Cycloketonization and linear polyketonization of alpha,omega-dicarboxylic acids. 7.Preparation and reactions of zinc salts of nonbranched dicarboxylic acids"**

(73) Patentinhaber: **HÜLS AKTIENGESELLSCHAFT Patentabteilung / PB 15 - Postfach 13 20 W-4370 Marl 1(DE)**

(72) Erfinder: **Kleine-Homann, Walter, Dr. Buchenallee 14 W-4408 Dülmen(DE)**

Rank Xerox (UK) Business Services

## Beschreibung

Die Synthese cyclischer Ketone wie Cycloheptanon (Suberon) ist umfassend in der Literatur beschrieben. Bei der sogenannten Dieckmann-Kondensation wird die Umsetzung eines Dicarbonsäureesters in Anwesenheit eines Alkalialkoholats vorgenommen. Das Ausbeutemaximum ist bei der Bildung von fünf- und sechsgliedrigen Ringen mit ca. 75 % zu erreichen. Der Einsatz längerkettiger Dicarbonsäureester (bis $C_{14}$) liefert dagegen bei diesem Verfahren nur geringe Ausbeuten (J. Org. Chem. 23 (1958) 1708).

Vergleichbare Ausbeuten werden auch bei der Cyclisierung von Adipinsäure zu Cyclopentanon erhalten. Als Kontakte sind insbesondere Zink-, Cadmium- und Mangancarbonat geeignet. Beim Einsatz von Korksäure kann jedoch lediglich eine Ausbeute an Cycloheptanon von ca. 40 % erzielt werden (Izv. Akad. SSR 1968 3 632 bis 636).

Ähnlich unbefriedigende Werte werden bei der sogenannten Tiffenaeu-Demjanov Ringerweiterung erhalten. Butadien und Acrylnitril werden zum 1-Cyanocyclohexen-3 umgesetzt, welches zum Amin reduziert anschließend diazotiert und zum Cycloheptanol umgesetzt wird. Durch Dehydrierung ist sodann das Cycloheptanon, das Suberon, zugänglich.

Bedeutend bessere Ausbeuten, bis ca. 95 %, erhält man bei der Ziegler-Kondensation. Diese Reaktion erfordert jedoch äquimolare Mengen an alkylierten Alkaliamiden in möglichst verdünnten Lösungen (Ann. Chem. 504 (1933) 95 bis 130).

Es besteht daher ein Interesse an einem Verfahren, das sowohl bei möglichst hohen Umsätzen und Selektivitäten als auch bei reduziertem Einsatz systemfremder Hilfsstoffe von leicht zugänglichen Ausgangsstoffen zum Cycloheptanon führt.

Die sich hieraus ergebende Aufgabe wird erfindungsgemäß entsprechend den Angaben der Patentansprüche gelöst.

Überraschenderweise ist die Synthese von Cycloheptanon (Suberon) aus Korksäureestern mit hohem Umsatz und hoher Selektivität in der Gasphase unter Verwendung von mit Zinkoxid dotierten Aluminiumoxid-Kontakten möglich.

Als Ester setzt man bevorzugt Korksäuredimethyl- oder Korksäuredibutylester oder auch deren Mischungen ein. Wegen ihres höheren Siedepunktes (größer 340 °C) sind Ester mit längerkettigen Alkylresten (größer $C_4$) in der Praxis weniger geeignet. Als Einsatzprodukt kann ein Reinprodukt sowie auch das direkt aus der Veresterung stammende Rohprodukt verwendet werden. Dabei ist es von Vorteil, wenn das Rohprodukt den zur Veresterung verwendeten Alkohol im Überschuß enthält. Sinngemäß kann ebenfalls ein anderer oben erwähnter Alkohol eingesetzt werden. Auch beim Einsatz des Reinproduktes führt der Zusatz obiger Alkohole zu einer Selektivitätssteigerung.

Die Dosierung des Alkohols ist dabei sowohl separat als auch im Gemisch mit dem Ester möglich. Das Molverhältnis Alkohol/Ester beträgt vorzugsweise 1 bis 20, insbesondere 3 bis 15.

Beim Einsatz des Rohproduktes aus der Veresterung läßt sich die Selektivität und auch die Wirtschaftlichkeit des Verfahrens steigern. Zur Erzielung kurzer Veresterungszeiten auch bei der Produktion des Dimethylesters sind dann keine Druckapparate erforderlich.

Beim Einsatz des Rohproduktes wird je nach Alkoholüberschuß in der Veresterungsstufe ein Umsatz von 90 bis 95 % angestrebt. Die nicht umgesetzte Korksäure bzw. der Halbester werden bei der Verdampfung überwiegend abgetrennt und zur Veresterung zurückgeführt.

Neben den beschriebenen Verdünnungen mit dem jeweiligen Alkohol ist auch Wasser zur Verdünnung geeignet. Zur weitestgehenden Vermeidung von Esterverseifungen sollte die Wassereinspeisung separat erfolgen. Bevorzugt gibt man das Wasser nach der Verdampfung der Korksäureester zu. Das 35 Molverhältnis Wasser/Ester beträgt vorzugsweise 1 bis 50, insbesondere 3 bis 20.

Eine weitere Möglichkeit zur Selektivitätssteigerung ist die Inertisierung z. B. mittels Stickstoff. Ein Nachteil der letzten Methode liegt darin, daß bei der Umgehung von Tiefstkühlungen durch Partialdampfdruck-Beladungen ein gewisser Verlust des wertvollen Endprodukts unumgänglich ist.

Als Katalysatoren sind mit Zink dotierte Kontakte geeignet. Als Trägermaterial ist Aluminiumoxid geeignet, beispielsweise handelsübliches Aluminiumoxid wie z. B. $Al_2O_3$ 3996 von der Firma Harshaw. Man dotiert das Aluminiumoxid vorzugsweise mit 5 bis 35 %, insbesondere mit 8 bis 20 % Metalloxid.

Zur Umsetzung verdampft man zunächst den Korksäureester und setzt ihn in der Gasphase an dem Katalysator um.

Die Umsetzung erfolgt bei Temperaturen von 300 bis 600 °C, bevorzugt von 400 bis 500 °C, wobei insbesondere bei Normaldruck gearbeitet wird. Wahlweise kann die Umsetzung sowohl bei Unterdruck als auch bei Überdruck erfolgen. Beim Einsatz längerkettiger Alkohole arbeitet man bevorzugt im Unterdruckbereich.

Die Umsetzung der Korksäure mit dem jeweiligen Alkohol erfolgt in bekannter Weise unter Einsatz

2

üblicher Katalysatoren wie Schwefelsäure, p-Toluolsulfonsäure oder Butyltitanat. Auch der Einsatz von handelsüblichen Ionentauscherharzen wie beispielsweise Lewatit SPC 108 oder SPC 118 ist als Katalysator möglich. Soll der reine Ester zum Einsatz gelangen, so wird das Rohprodukt fraktioniert. Bei Verwendung des Rohproduktes als Einsatzstoff wird in den Fällen, wo als Veresterungskatalysator ein Ionentauscherharz verwendet war, dieses zunächst abfiltriert und zur Vermeidung von Produktverlusten mit Alkohol nachgewaschen.

Der bei der Verdampfung des Rohproduktes verbleibende Rückstand, der vorwiegend aus dem Halbester besteht, kann zur nächsten Veresterung wieder eingesetzt werden.

Die Aufarbeitung des rohen Cycloheptanon (Suberon) erfolgt vorzugsweise destillativ. Wurde u. a. Wasser in den Reaktor dosiert, so erfolgt die Aufarbeitung vorteilhafterweise zweistufig. Das zweiphasige Gemisch wird getrennt. Unter Einbeziehung des in der wäßrigen Phase gelösten Cycloheptanon (Suberon) werden die organischen Bestandteile der Wasserphase durch Auskreisen des Wasser separiert.
Nach Vereinigung beider organischer Phasen werden sie destillativ aufgearbeitet.

Man kann die separate Aufarbeitung der Wasserphase auch umgehen. Von Nachteil ist bei der einstufigen destillativen Aufarbeitung jedoch die längere thermische Belastung des Gesamtaustrags.

Bei der erfindungsgemäßen Umsetzung erreicht man Umsätze von 60 bis 95 % und Selektivitäten von 50 bis 80 %. Das Cycloheptanon (Suberon) setzt man insbesondere als Riechstoff oder pharmazeutisches Zwischenprodukt ein.

Beispiele

a) Veresterung
In einem Rührkolben mit aufgesetzter Kolonne und Wasserauskreiser wird Korksäure mit dem Alkohol im Verhältnis 1 : 1 vorgelegt. Nach Zugabe des Katalysators wird bis zum Rückfluß erwärmt. Bei Ausbildung einer Wasserphase, beispielsweise beim Einsatz von Butanol, trennt man zur Verkürzung der Reaktionszeit das Wasser ab. Nach Erreichen des gewünschten Umsatzes wird bei Verwendung eines Ionentauschers dieser zunächst abgetrennt und mit etwas Alkohol nachgewaschen. Das Filtrat wird dann ebenso wie bei Verwendung von z. B. p-Toluolsulfonsäure als Katalysator mit Natronlauge neutral gestellt.

Flüssige Rückstände, die bei der Verdampfung des Rohproduktes im Bereich des Reaktors anfallen, werden direkt zur Veresterung mit eingesetzt.

b) Umsetzung zum Cycloheptanon (Suberon)
1 l des jeweiligen Kontaktes wird in einem elektrisch beheizbaren Quarzrohr von 1 m Länge und 50 mm Durchmesser vorgelegt. Während der Aufheizphase bis zur gewünschten Reaktionstemperatur wird der Reaktor mit Stickstoff durchströmt. Die Temperaturerfassung erfolgt mit Hilfe eines Widerstandsthermometers, das sich in einem zentrierten Quarzrohr von 6 mm Durchmesser befindet und dort je nach Anforderung axial in die gewünschte Position verschoben werden kann.

Die flüssigen Einsatzprodukte werden über einen Verdampfer mit nach geschaltetem Überhitzer auf die erforderliche Reaktionstemperatur erhitzt, um danach auf das Katalysatorbett geleitet zu werden. Die Zudosierung des Alkohols bzw. des Wassers erfolgte nach dem Verdampfer jedoch vor dem Überhitzer.

c) Die Aufarbeitung des Rohaustrages der Suberonherstellung erfolgt destillativ an einer 0,5 m Kolonne, die mit Gewebepackungen ausgerüstet war.

Bildet sich eine wäßrige Phase im Rohprodukt aus, so wird diese zunächst abgetrennt und separat mit Hilfe eines Wasserauskreisers aufgearbeitet. Die dabei anfallende organische Phase wird mit der des Reaktoraustrages vereinigt und gemeinsam aufgearbeitet. Die Reinheit des gewünschten Cycloheptanons (Suberon) als Hauptlauf ist 99 %.

Wird die Wasserphase nicht separat behandelt, sondern aus dem zweiphasigen Reaktoraustrag direkt ausgekreist, so kann es je nach Destillationsbedingungen zu partiellen Rückspaltungen des nicht umgesetzten Esteranteils kommen.

Beispiele 1 bis 4

Nachfolgend sind die Werte aufgelistet, die sich bei der Zudosierung des korrenspondierenden Alkohols ergeben:

| Beispiel | Kontakt | Korksäure-ester | Molverh. Alkohol/Ester | Reaktions-temperatur (°C) | Umsatz (%) | Selekti-vität (%) |
|---|---|---|---|---|---|---|
| 1 | A | I | 15 | 420 | 59 | 63 |

In der nachfolgenden Tabelle sind exemplarisch die Werte aufgelistet, die sich bei der Zudosierung von Wasser ergeben:

| Beispiel | Kontakt | Korksäure-ester | Molverh. Alkohol/Ester | Reaktions-temperatur (°C) | Umsatz (%) | Selekti-vität (%) |
|---|---|---|---|---|---|---|
| 2 | A | I | 20 | 440 | 65 | 76 |
| 3 | B | I | 3 | 460 | 82 | 61 |
| 4 | A | II | 6 | 450 | 82 | 72 |

Vergleichsbeispiel

Korksäuredimethylester wird ohne Verdünnung bei 420 °C am Kontakt I zum Cycloheptanon (Suberon) umgesetzt. Nach der Destillation des Rohaustrages wird ein Hauptlauf mit einer Reinheit von 99,2 % isoliert. Bei einem Umsatz von 74 % ergibt sich eine Selektivität von 38 %.

Abkürzungen:

Ester I:      Korksäuredimethylester
Ester II:     Korksäuredibutylester
Kontakt A:    12 % ZnO auf $Al_2O_3$
Kontakt B:    18 % ZnO auf $Al_2O_3$

**Patentansprüche**

1. Verfahren zur Herstellung von Cycloheptanon aus Korksäureestern,
   dadurch gekennzeichnet,
   daß man Korksäuredimethylester oder Korksäuredibutylester verdampft und in alkoholischer Verdünnung, wobei das Molverhältnis Alkohol/Ester von 1 bis 20, vorzugsweise 3 bis 15, beträgt und/oder wäßriger Verdünnung, wobei das Molverhältnis Wasser/Ester von 1 bis 20, vorzugsweise 3 bis 20, beträgt in der Gasphase an mit Zinkoxid dotierten Aluminiumoxid-Kontakten bei Temperaturen von 300 bis 600 °C umsetzt.

2. Verfahren nach Anspruch 1,
   dadurch gekennzeichnet,
   daß man als Alkohole $C_1$-$C_4$-Alkohole einsetzt.

3. Verfahren nach einem der Ansprüche 1 bis 2,
   dadurch gekennzeichnet,
   daß man das Wasser nach der Verdampfung der Korksäureester zugibt.

4. Verfahren nach einem der Ansprüche 1 bis 3,
   dadurch gekennzeichnet,
   daß man die Umsetzung bei Temperaturen von 400 bis 500 °C durchführt.

**5.** Verfahren nach einem der Ansprüche 1 bis 4,
dadurch gekennzeichnet, daß man die Umsetzung an Katalysatoren durchführt, die 5 bis 35, vorzugsweise 8 bis 20 %, Zinkoxid auf Aluminiumoxid enthalten.

## Claims

**1.** A Process for the preparation of cycloheptanone from esters of suberic acid, characterised in that dimethyl suberate or dibutyl suberate is evaporated and reacted in the gas phase on aluminium oxide contact catalysts doped with zinc oxide at temperatures of 300 to 600°C in an alcoholic dilution, the molar ratio of alcohol/ester being from 1 to 20, preferably 3 to 15, and/or in an aqueous dilution, the molar ratio of water/ ester being from 1 to 20, preferably 3 to 20.

**2.** A process according to claim 1, characterised in that $C_1$-$C_4$-alcohols are employed as alcohols.

**3.** A process according to either of claims 1 and 2, characterised in that the water is added after the evaporation of the ester of suberic acid.

**4.** A process according to any of claims 1 to 3. characterised in that the reaction is carried out at temperatures from 400 to 500°C.

**5.** A process according to any of claims 1 to 4, characterised in that the reaction is carried out on catalysts which contain 5 to 35%, preferably 8 to 20%, of zinc oxide on aluminium oxide.

## Revendications

**1.** Procédé de préparation de cycloheptanone à partir d'esters de l'acide subérique,
caractérisé par le fait que l'on concentre le subérate de diméthyle ou le subérate de dibutyle et qu'en dilution alcoolique, le rapport molaire alcool/ester étant de 1 à 20, de préférence de 3 à 15, et/ou en dilution aqueuse, le rapport molaire eau/ester étant de 1 à 20, de préférence de 3 à 20, on le fait réagir en phase gazeuse, à des températures de 300 à 600°C, sur des catalyseurs à l'oxyde d'aluminium additionnés d'oxyde de zinc.

**2.** Procédé selon la revendication 1,
caractérisé par le fait que l'on utilise comme alcools des alcools comportant de 1 à 4 atomes de carbone.

**3.** Procédé selon l'une des revendications 1 et 2,
caractérisé que l'on ajoute de l'eau après la concentration des esters de l' acide subérique.

**4.** Procédé selon l'une des revendications 1 à 3,
caractérisé par le fait que l'on effectue la réaction à des températures de 400 à 500°C.

**5.** Procédé selon l'une des revendications 1 à 4,
caractérisé par le fait que l'on effectue la réaction sur des catalyseurs renfermant de 5 à 35, de préférence de 8 à 20 % d'oxyde de zinc sur de l'oxyde d'aluminium.